# EUROPEAN PATENT SPECIFICATION

(11) **EP 2 937 052 B1**
(45) Date of publication and mention of the grant of the patent: **13.03.2019**
(21) Application number: 13865082.5
(22) Date of filing: 21.11.2013
(51) Int. Cl.: A61B 18/04, A61B 18/12, A61B 18/00

(54) **LOW-TEMPERATURE PLASMA GENERATOR USED IN SURGERY**
NIEDERTEMPERATURPLASMAGENERATOR VERWENDET IN DER CHIRURGIE
GÉNÉRATEUR DE PLASMA BASSE TEMPÉRATURE UTILISÉ EN CHIRURGIE

(30) Priority: 21.12.2012 CN 201210561325
(43) Date of publication of application: 28.10.2015
(73) Proprietor: Chengdu Mechan Electronic Technology Co., Ltd., Chengdu, Sichuan 611130 (CN)
(72) Inventor: LI, Zheng, Chengdu Sichuan 610000 (CN); HE, Chengdong, Chengdu Sichuan 610000 (CN)
(74) Representative: Cabinet Chaillot
(86) International application number: PCT/CN2013/001425
(87) International publication number: WO 2014/094352

(56) References cited:
- CN-A- 1 491 620
- CN-A- 103 040 519
- CN-U- 201 727 580
- CN-U- 202 173 470
- CN-U- 202 173 470
- GB-A- 2 454 461
- GB-A- 2 486 343
- US-A1- 2011 028 970
- US-A1- 2011 028 970

## Description

### Technical Field

The invention relates to a plasma generator, and specifically relates to a low-temperature plasma generator used in surgeries and a probe system composed by the same.

### Background Technology

A surgery is one of the principal therapies of Western medicine and is commonly known as "perform an operation", with the primary aims at removing the pathological changes, repairing injuries, transplanting organs, and improving functions and morphologies of the organism. Early surgeries are restricted to simple manual methods to slice, cut, suture in human body surface, such as abscess drainage, tumor resection, traumatic suture, etc. Hence, a surgery is an operating mode that damages the tissue completeness (cutting) or recovers the tissue damaged in completeness. In nowadays society, with the development of scientific technologies, plasma technology has been widely used in surgeries and has made a significant contribution to smooth surgeries. However, over-high temperature, which can be easily produced by existing laser probes, may result in damages to healthy tissues of a patient during the surgery, and hemostasis and cleaning are performed by other auxiliary apparatus to the healthy tissues that are damaged, thus the workload of the medical staff being increased and life safety of the patient being affected. Moreover, the over-high temperature may also result in volatilization of solid and liquid and thus smoke is produced, which greatly impacts the smooth running of a surgery and endangers the patient's life in severe cases. Therefore, it is urgent to work out a surgical apparatus to overcome the above drawbacks. CN-U-202173470 discloses an electric surgical bipolar plasma generator. GB-A-2486343 discloses a system configured to produce hot and cool plasma for surface coagulation and tissue sterilisation respectively.

### Contents of the Invention

The purpose of the invention is to provide a low-temperature plasma generator used in surgeries and a probe system composed by the same to overcome the drawbacks that the healthy skin of a patient be damaged by over-high temperature easily produced by a laser probe and the smoke production during the cutting and ablation process by existing surgeries.

In order to achieve the above purposes, the technical solution used in the invention is as follows:
A low-temperature plasma generator used in surgeries, characterized in that the low-temperature plasma generator comprises a CPU control module, a vibration module which is connected to the CPU control module, and an output module which is connected to the vibration module; wherein the CPU control module comprises:
a microprocessor;
an analogue-to-digital conversion circuit that is connected to an input end of the microprocessor;
an interface chip which is interacted with the microprocessor for signal interaction;
and a display driving circuit and a voice prompting device that are electrically connected to an output end of the interface chip, respectively,
and a display device for receiving the output signals of the display driving circuit;
the analogue-to-digital conversion circuit and the microprocessor being electrically connected to the vibration module, respectively.

Further, the vibration module comprises:
a key operation unit for inputting signals to the microprocessor;
a current detection circuit for inputting detecting signals to the analogue-to-digital conversion circuit;
a power control circuit for receiving the control signals of the microprocessor;
and a first vibration unit and a voltage amplification circuit that are connected to the output end of the power control circuit, respectively, the first vibration unit and the voltage amplification circuit being connected bi-directionally and being both connected to the output module;
and a second vibration unit for sending signals to the voltage amplification circuit.

Still further, the output module comprises:
a first power amplification circuit, the input end of which is connected to the first vibration unit;
a second power amplification circuit, the input end of which is connected to the voltage amplification circuit and which inputs detecting signals to the current detection circuit.

On the basis of the above technologies, a probe system composed of the above low-temperature plasma generator is also provided according to the invention, and it comprises a normal saline input apparatus and a plasma probe, and a low-temperature plasma generator which is connected to the plasma probe; the low-temperature plasma generator comprises a CPU control module, a vibration module which is connected to the CPU control module, and an output module which is connected to the vibration module; wherein the CPU control module comprises:
a microprocessor;
an analogue-to-digital conversion circuit that is connected to an input end of the microprocessor;
an interface chip which is interacted with the microprocessor for signal interaction;
and a display driving circuit and a voice prompting device that are electrically connected to an output end of the interface chip, respectively, and a display device for receiving the output signals of the display driving circuit;
the analogue-to-digital conversion circuit and the microprocessor being electrically connected to the vibration module, respectively.

Among other things, the vibration module comprises:
a key operation unit for inputting signals to the microprocessor;
a current detection circuit for inputting detecting signals to the analogue-to-digital conversion circuit;
a power control circuit for receiving the control signals of the microprocessor;
and a first vibration unit and a voltage amplification circuit that are connected to the output end of the power control circuit, respectively, the first vibration unit and the voltage amplification circuit being connected bi-directionally and being both connected to the output module;
and a second vibration unit for sending signals to the voltage amplification circuit.

Moreover, the output module comprises:
a first power amplification circuit, the input end of which is connected to the first vibration unit;
a second power amplification circuit, the input end of which is connected to the voltage amplification circuit and which inputs detecting signals to the current detection circuit.

Further, the plasma probe comprises a tool bit and a handle which are fixed as a whole, wherein:
the handle comprises a handle body, an electrode input interface which is provided on the handle body and is connected to the output end of the first power amplification circuit and the second power amplification circuit, respectively, and a normal saline input interface which is provided on one end of the handle body and a waste liquid discharge port located on the same end of the handle body with the normal saline input interface; the tool bit comprises a tool bit body that is fixed as a whole with the handle body, an electrode provided on the tool bit body and connected to the electrode input interface, the electrode comprising a first end of the electrode provided on tool bit body, and a second end of the electrode provided at the end that is far away from the handle body on the tool bit, and a normal saline output hole provided on the tool bit body and interlinked with the normal saline input interface, and a waste liquid absorption hole provided at the end that is far away from the handle on the tool bit body and interlinked with the waste liquid discharge port.

Still further, the normal saline input apparatus comprises a normal saline storage bottle, a transfer tube, one end of which is provided on the normal saline storage bottle and interlinked therewith; and a normal saline input controller provided on the transfer tube, wherein the other end of the transfer tube is connected to the normal saline input interface.

Compared with the prior art, the invention has the following advantages:
1. The plasma probe is controlled by the low-temperature plasma generator according to the invention, and electric energy is excited by the same. Plasma is generated when the normal saline flows to the tool bit. Because a lot of heat is taken away when the normal saline flows, tissues are cut at low temperature to achieve low-temperature cut, hemostasis and ablation. Due to the cooling effect of the normal saline, neither the problem that smoke is produced through volatilization of solid and liquid resulting from over-high temperature nor the problem that the healthy skin around the cutting of a patient may be damaged will occur. The problems that a normal surgery is affected by smoke produced through volatilization of solid and liquid, and the problem that healthy skin around the cutting may be damaged due to over-high temperature are effectively solved.
2. A waste liquid absorption hole is provided on the tool bit according to the invention, through which waste tissues, tissue fluid, and needless normal saline produced during ablation and cutting can be absorbed in time to avoid healthy skin of a patient from being influenced by the diseased tissues and being infected. Meanwhile, because cutting and ablation during a surgery may result in bleeding, and blood can be absorbed and cleaned by the waste liquid absorption hole instead of other auxiliary devices, not only the workload of the medical staff can be decreased, the surgery process can be improved, but also the medical materials can be saved, and the cost of a surgery and economic burden of a patient can be reduced.
3. The plasma probe according to the invention is used instead of a conventional surgical probe, and overcome the drawbacks of the inconvenience in operating a conventional surgical probe and the inflexibility in applying the conventional surgical probe at privileged sites being overcome. Cutting and ablation can be flexibly performed at whatever site of a patient, and surgical results can be ensured.
4. The plasma probe is improved in both the control circuit aspect and the physical structure aspect according to the invention, temperature control of the plasma probe can be more precise, the physical structure can be more reasonable, and the practical value can be greatly enhanced.

### Brief Description of the Drawings

Figure 1 shows a schematic block diagram of the low-temperature plasma generator according to the invention;
Figure 2 shows a structure diagram of one embodiment of the invention; and
Figure 3 shows an amplified structure schematic diagram of the tool bit in one embodiment of the invention.

Components referred to the reference numerals of the drawings are as follows:
1-normal saline storage bottle, 2-transfer tube, 3-normal saline input controller, 4-bracket, 5-low-temperature plasma generator, 6-waste liquid discharge tube, 7-handle body, 8-tool bit body, 9-the first end of the electrode, 10-normal saline output hole, 11-waste liquid absorption hole,
12-the second end of the electrode.

### Detailed Embodiments

The invention will be further illustrated with the combination of the drawings and the embodiments. The embodiments of the invention comprises but not limited to the following embodiments.

### Embodiments

The subject matter of the invention comprises two parts: a low-temperature plasma generator, and a probe system composed by the same, wherein the low-temperature plasma generator is primarily used in generating plasma in normal saline to provide energy, and the probe system is a complete plasma surgical probe system, which can be used for cutting and ablation in various surgeries.

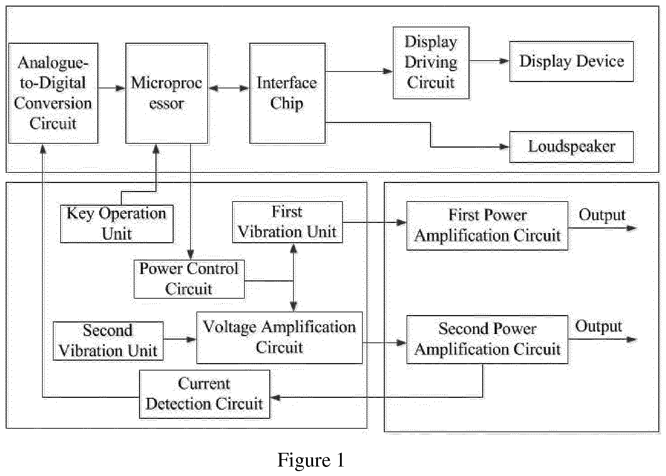

As shown in Figure 1, the low-temperature plasma generator primarily comprises three parts: a CPU control module, a vibration module and an output module, wherein the CPU module is used for controlling and prompting, and the prompting comprises display and voice prompting.

Specifically, the CPU module comprises a microprocessor; an analogue-to-digital conversion circuit, the output end of which is connected to the microprocessor; and an interface chip which is interacted with the microprocessor. Among these, the interface chip transmits a signal to a display driving circuit and a voice prompting device, the display driving circuit transmits the signal to a display device after receiving the same, and the display device displays the information; the analogue-to-digital conversion circuit receives the signal transmitted by the vibration module.

Preferably, a liquid crystal display is chosen as the display device, and a loudspeaker is chosen as the voice prompting device in the embodiment.

The vibration module comprises a current detection circuit which transmits a signal to the analogue-to-digital conversion circuit; a key operation unit which transmits a signal to the microprocessor; and a power control circuit which receives a signal from the microprocessor. The power control circuit transmits a control signal from the microprocessor simultaneously to a first vibration unit and a voltage amplification circuit that are connected bi-directionally, and the control signal is output by the first vibration unit and the voltage amplification circuit so as to control the plasma probe precisely. In order to ensure the effect, a vibrating signal is also input through a second vibration unit by the voltage amplification circuit to ensure the availability of the control signal.

The output module receives a signal from the first vibration unit and the voltage amplification circuit respectively, and outputs the control signal through a first power amplification circuit and a second power amplification circuit. Moreover, the second power amplification circuit feeds back a signal to the current detection circuit while outputs a signal, and the signal is transmitted back to the microprocessor via the analogue-to-digital conversion circuit to achieve the monitoring of the output and ensure the safety and reliability.

The working process of the above plasma generator is as follows:
Electrical power is input to the low-temperature plasma generator to produce power driving current and system display control current. At this moment, the display, control, power regulation, voice output and alarm of the whole system are all processed and controlled at a high speed by the microprocessor.

First of all, a required power output value is preset by the microprocessor and output to the vibration module, the power output value is converted into a pulse width modulation signal by the vibration module, a first-level power driving is achieved under the control of the first power amplification circuit, and the pulse width modulation signal is output. Then a driving and amplification of second-level power are achieved by the second power amplification circuit, and plasma energy is output. Because the second power amplification circuit enters the CPU control module again through the vibration module, the amount and variation of driving energy can also be monitored at a high speed by the low-temperature plasma generator during power amplification process, to avoid overcurrent and overvoltage generated during the amplification process that affect the safety of an apparatus and endanger the patient's life.

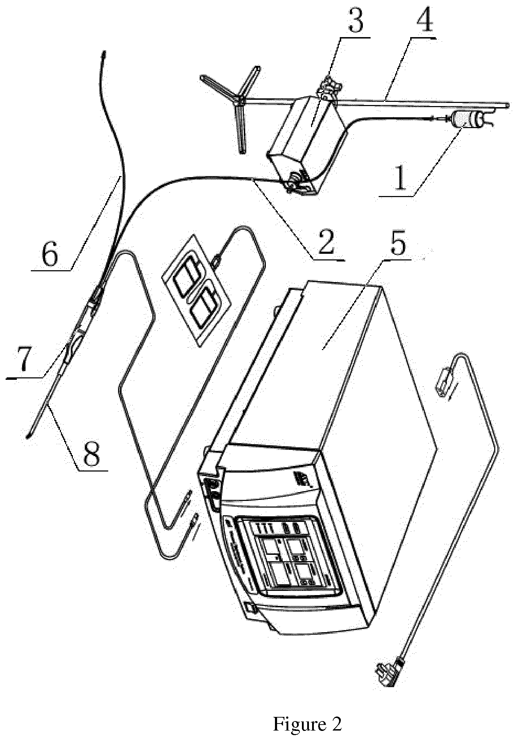

As shown in Figure 2, a probe system is also provided by the invention and comprises a normal saline input apparatus and a plasma probe, and a low-temperature plasma generator connected to the plasma probe.

The normal saline input apparatus comprises a normal saline storage bottle 1, a transfer tube 2 connected to the interface of the normal saline storage bottle, and a normal saline input controller 3 for controlling the transportation of the normal saline with one of its end provided on the transfer tube 2. The transfer tube is used for transferring the normal saline that is stored in normal saline storage bottle to the plasma probe, and the transfer tube is controlled by the normal saline input controller to adjust the flow of the normal saline.

A bracket 4 is also provided in the embodiment, and a hook is arranged on the upper end of the bracket 4 for hanging up the normal saline storage bottle, and the normal saline input controller is fixed on the bracket 4.

The plasma probe comprises a tool bit and a handle which are fixed as a whole. The handle comprises a handle body 7, wherein an electrode input interface, a normal saline input interface and a waste liquid discharge port are provided in the handle body and are connected with the low-temperature plasma generator. The normal saline input interface and the waste liquid discharge port are both arranged at the end of the handle body that is far away from tool bit on the handle body, a waste liquid discharge tube 6 is connected to the waste liquid discharge hole for discharging the waste produced in cutting and ablation during a surgery, and the transfer tube 2 is connected to the normal saline input interface for receiving the normal saline output by the transfer tube.

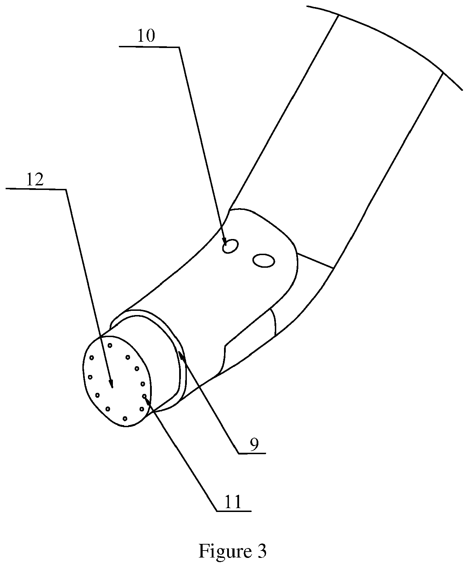

As shown in Figure 3, the tool bit comprises a tool bit body 8 that is fixed as a whole with the handle body, an electrode, a normal saline output hole 10 provided on the tool bit body and interlinked with the normal saline input interface, and a waste liquid absorption hole 11 arranged at the end of the tool bit body that is far away from the handle and interlinked with the waste liquid discharge port, wherein the electrode comprises a first end 9 of the electrode and a second end 12 of the electrode. One end of the electrode is arranged on the tool bit and the other end is arranged at the end of the tool bit that is far away from the handle body.

The normal saline enters the tool bit body from the handle body and the waste liquid discharges from the tool bit body to the handle body, hence a normal saline passage and a waste liquid passage are arranged inside the plasma probe; the normal saline input interface is interlinked with the normal saline output hole via the normal saline passage for outputting the normal saline through the normal saline output hole, while the waste liquid discharge hole is interlinked with the waste liquid absorption hole via the waste liquid passage for absorbing the waste liquid and cutting and ablating the diseased tissue.

The low-temperature plasma generator is connected with the handle of the plasma probe via the electrode input interface, and the method for controlling the plasma probe is as follows:
Step 1, using the low-temperature plasma generator to generate the plasma, on one hand, by outputting a driving signal from the first power amplification circuit, on the other hand, by outputting the plasma energy from the second power amplification circuit, and generating two electrodes being input to the tool bit, a positive electrode and a negative electrode being formed at the first end and the second end of the electrode on the tool bit, respectively;
Step 2, using the normal saline output controller to extract the normal saline from the normal saline storage bottle, and guiding the normal saline to flow towards the plasma tool bit;
Step 3, guiding the normal saline to flow towards the first end and the second end of the electrode, thus the first end and the second end of the electrode are conducted because of the electrical conductivity of the normal saline, and the low-temperature plasma being generated at the second end of the electrode when it is conducted, thus " a plasma probe" being formed for performing the cutting and ablation in the body; meanwhile, the waste tissue, tissue fluid, blood and needless normal saline produced during ablation and cutting can be absorbed by the waste liquid absorption hole.

It has to be mentioned that each circuit, such as an analogue-to-digital conversion circuit, a display driving circuit, a power control circuit, a voltage amplification circuit, a current detection circuit, the first power amplification circuit and the second power amplification circuit, can be embodied as existing circuit and other hardware apparatus can be also embodied as existing apparatus that are need not to be repeated here.

As mentioned above, the present invention can be implemented well.

## Claims

1. A low-temperature plasma generator for use in surgeries, wherein: the low-temperature plasma generator comprises a CPU control module, a vibration module which is connected to the CPU control module, and an output module which is connected to the vibration module; wherein the CPU control module comprises:
a microprocessor;
an analogue-to-digital conversion circuit that is connected to an input end of the microprocessor;
an interface chip which is interacted with the microprocessor for signal interaction;
and a display driving circuit and a voice prompting device that are respectively electrically connected to an output end of the interface chip, and a display device for receiving the output signals of the display driving circuit;
the analogue-to-digital conversion circuit and the microprocessor being respectively electrically connected to the vibration module;
wherein the vibration module comprises:
a key operation unit for inputting signals to the microprocessor;
a current detection circuit for inputting detecting signals to the analogue-to-digital conversion circuit;
a power control circuit for receiving the control signals of the microprocessor;
and a first vibration unit and a voltage amplification circuit which are respectively connected to the output end of the power control circuit, the first vibration unit and the voltage amplification circuit being connected bi-directionally and being both connected to the output module;
and a second vibration unit for sending signals to the voltage amplification circuit.

2. The low-temperature plasma generator used in surgeries according to claim 1, **characterized in that** the output module comprises:
a first power amplification circuit, the input end of which is connected to the first vibration unit;
a second power amplification circuit, the input end of which is connected to the voltage amplification circuit and which inputs detecting signals to the current detection circuit.

3. A probe system composed of a low-temperature plasma generator, **characterized in that** the probe system comprises a normal saline input apparatus and a plasma probe, and a low-temperature plasma generator as claimed in one of the preceding claims which is connected to the plasma probe.

4. The probe system composed of a low-temperature plasma generator according to claim 3, **characterized in that** the output module comprises:
a first power amplification circuit, the input end of which is connected to the first vibration unit;
a second power amplification circuit, the input end of which is connected to the voltage amplification circuit and which inputs detecting signals to the current detection circuit.

5. The probe system composed of a low-temperature plasma generator according to claim 4, **characterized in that** the plasma probe comprises a tool bit and a handle which are fixed as a whole, wherein:
the handle comprises a handle body (7), an electrode input interface which is provided on the handle body (7) and is connected to the output end of the first power amplification circuit and the second power amplification circuit, respectively, and a normal saline input interface which is provided on one end of the handle body (7) and a waste liquid discharge port located on the same end of the handle body (7) with the normal saline input interface;
the tool bit comprises a tool bit body (8) that is fixed as a whole with the handle body (7), an electrode provided on the tool bit body (8) and connected to the electrode input interface, the electrode comprising a first end (9) of the electrode provided on tool bit body (8), and a second end (12) of the electrode provided at the end that is far away from the handle body (7) on the tool bit, and a normal saline output hole (10) provided on the tool bit body (8) and interlinked with the normal saline input interface, and a waste liquid absorption hole (11) provided at the end that is far away from the handle on the tool bit body (8) and interlinked with the waste liquid discharge port.

6. The probe system composed of a low-temperature plasma generator according to claim 5, **characterized in that** the normal saline input apparatus comprises a normal saline storage bottle (1); a transfer tube (2), one end of which is provided on the normal saline storage bottle (1) and interlinked therewith; and a normal saline input controller (3) provided on the transfer tube (2), wherein the other end of the transfer tube (2) is connected to the normal saline input interface.

## Patentansprüche

1. Niedertemperaturplasmagenerator, verwendet in der Chirurgie, wobei der Niedertemperaturplasmagenerator ein CPU-Steuermodul, ein Vibrationsmodul, das mit dem CPU-Steuermodul verbunden ist, und ein Ausgangsmodul umfasst, das mit dem Vibrationsmodul verbunden ist, wobei das CPU-Steuermodul Folgendes umfasst:
einen Mikroprozessor;
eine Analog-Digital-Umwandlungsschaltung, die mit einem Eingangsende des Mikroprozessors verbunden ist;
einen Schnittstellenchip, der mit dem Mikroprozessor zur Signalinteraktion interagiert,
und eine Displaytreiberschaltung und eine Sprachansagevorrichtung, die jeweils elektrisch an ein Ausgangsende des Schnittstellenchips verbunden sind, und eine Displayvorrichtung, um die Ausgangssignale der Displaytreiberschaltung zu empfangen;
wobei die Analog-Digital-Umwandlungsschaltung und der Mikroprozessor jeweils elektrisch an das Vibrationsmodul verbunden sind;
wobei das Vibrationsmodul Folgendes umfasst:
eine Schlüsselbetriebseinheit, um Signale in den Mikroprozessor einzugeben;
eine Stromnachweischaltung, um Nachweissignale in die Analog-Digital-Umwandlungsschaltung einzugeben;
eine Leistungssteuerschaltung, um die Steuersignale des Mikroprozessors zu empfangen;
und eine erste Vibrationseinheit und eine Spannungsverstärkungsschaltung, die jeweils mit dem Ausgangsende der Leistungssteuerschaltung verbunden sind, wobei die erste Vibrationseinheit und die Spannungsverstärkungsschaltung bidirektional verbunden und beide an das Ausgangsmodul verbunden sind;
und eine zweite Vibrationseinheit, um Signale an die Spannungsverstärkungsschaltung zu schicken.

2. Niedertemperaturplasmagenerator, verwendet in der Chirurgie, nach Anspruch 1, **dadurch gekennzeichnet, dass** das Ausgangsmodul Folgendes umfasst:
eine erste Leistungverstärkungsschaltung, deren Eingangsende mit der ersten Vibrationseinheit verbunden ist,
eine zweite Leistungverstärkungsschaltung, deren Eingangsende mit der Spannungsverstärkungsschaltung verbunden ist, und die die Nachweissignale in die Stromnachweisschaltung eingibt.

3. Sondensystem, zusammengesetzt aus einem Niedertemperaturplasmagenerator, **dadurch gekennzeichnet, dass** das Sondensystem eine Vorrichtung zum Eingang von physiologischer Kochsalzlösung und eine Plasmasonde und einen Niedertemperaturplasmagenerator nach einem der vorhergehenden Ansprüche umfasst, der mit der Plasmasonde verbunden ist.

4. Sondensystem, zusammengesetzt aus einem Niedertemperaturplasmagenerator nach Anspruch 3, **dadurch gekennzeichnet, dass** das Ausgangsmodul Folgendes umfasst:
eine erste Leistungverstärkungsschaltung, deren Eingangsende mit der ersten Vibrationseinheit verbunden ist,
eine zweite Leistungverstärkungsschaltung, deren Eingangsende mit der Spannungsverstärkungsschaltung verbunden ist, und die die Nachweissignale in die Stromnachweisschaltung eingibt.

5. Sondensystem, zusammengesetzt aus einem Niedertemperaturplasmagenerator nach Anspruch 4, **dadurch gekennzeichnet, dass** die Plasmasonde einen Werkzeugeinsatz und einen Griff umfasst, die als ein Ganzes fixiert sind, wobei
der Griff einen Griffkörper (7), eine Elektrodeneingangsschnittstelle, die auf dem Griffkörper (7) bereitgestellt und mit dem Ausgangsende der ersten Leistungsverstärkungsschaltung bzw. der zweiten Leistungsverstärkungsschaltung verbunden ist, und eine Schnittstelle zum Eingang von physiologischer Kochsalzlösung, die auf einem Ende des Griffkörpers (7) bereitgestellt ist, und einen Flüssigabfall-Entladungsanschluss, der sich auf dem gleichen Ende des Griffkörpers (7) mit der Eingangsschnittstelle für physiologische Kochsalzlösung befindet, umfasst,
wobei der Werkzeugeinsatz einen Werkzeugeinsatzkörper (8), der als ein Ganzes mit den Griffkörper (7) fixiert ist, eine Elektrode, die auf dem Werkzeugeinsatzkörper (8) bereitgestellt und mit der Elektrodeneingangsschnittstelle verbunden ist, umfasst, wobei die Elektrode ein erstes Ende (9) der Elektrode, das auf dem Werkzeugeinsatzkörper (8) bereitgestellt ist, und eine zweites Ende (12) der Elektrode, das auf dem Ende bereitgestellt ist, das weit entfernt vom Griffkörper (7) auf dem Werkzeugeinsatz ist, und ein Ausgangsloch für physiologische Kochsalzlösung (10), das auf dem Werkzeugeinsatzkörper (8) bereitgestellt und mit der Eingangsschnittstelle von physiologischer Kochsalzlösung gekoppelt ist, und ein Flüssigabfall-Absorptionsloch (11) umfasst, das auf dem Ende bereitgestellt ist, das weit entfernt vom Griff auf dem Werkzeugeinsatzkörper (8) ist und mit dem Flüssigabfall-Entladungsanschluss gekoppelt ist.

6. Sondensystem, zusammengesetzt aus einem Niedertemperaturplasmagenerator nach Anspruch 5, **dadurch gekennzeichnet, dass** die Eingangsvorrichtung von physiologischer Kochsalzlösung eine Speicherflasche für physiologische Kochsalzlösung (1); ein Übertragungsrohr (2), von dem ein Ende auf der Speicherflasche für physiologische Kochsalzlösung (1) bereitgestellt und damit gekoppelt ist; und eine Eintrittssteuerung für physiologische Kochsalzlösung (3), die auf dem Übertragungsrohr (2) bereitgestellt ist, umfasst, wobei das andere Ende des Übertragungsrohrs (2) mit der Eingangsschnittstelle für physiologische Kochsalzlösung verbunden ist.

## Revendications

1. Générateur de plasma basse température pour une utilisation en chirurgie, dans lequel : le générateur de plasma basse température comprend un module de commande à UC, un module de vibration qui est connecté au module de commande à UC, et un module de sortie qui est connecté au module de vibration ; le module de commande à UC comprenant :
un microprocesseur ;
un circuit de conversion analogique/numérique qui est connecté à une extrémité d'entrée du microprocesseur ;
une puce d'interface qui interagit avec le microprocesseur pour une interaction de signal ; et
un circuit de pilotage d'affichage et un dispositif de guide vocal qui sont respectivement connectés électriquement à une extrémité de sortie de la puce d'interface, et un dispositif d'affichage pour recevoir les signaux de sortie du circuit de pilotage d'affichage ;
le circuit de conversion analogique/numérique et le microprocesseur étant respectivement connectés électriquement au module de vibration ;
le module de vibration comprenant :
une unité d'actionnement à touche pour entrer des signaux dans le microprocesseur ;
un circuit de détection de courant pour entrer des signaux de détection dans le circuit de conversion analogique/numérique ;
un circuit de commande de puissance pour recevoir les signaux de commande du microprocesseur ; et
une première unité de vibration et un circuit d'amplification de tension qui sont respectivement connectés à l'extrémité de sortie du circuit de commande de puissance, la première unité de vibration et le circuit d'amplification de tension étant connectés de manière bidirectionnelle et tous deux connectés au module de sortie ; et
une seconde unité de vibration pour envoyer des signaux au circuit d'amplification de tension.

2. Générateur de plasma basse température utilisé en chirurgie selon la revendication 1, **caractérisé par le fait que** le module de sortie comprend :
un premier circuit d'amplification de puissance, dont l'extrémité d'entrée est connectée à la première unité de vibration ;
un second circuit d'amplification de puissance, dont l'extrémité d'entrée est connectée au circuit d'amplification de tension et qui entre des signaux de détection dans le circuit de détection de courant.

3. Système de sonde composé d'un générateur de plasma basse température, **caractérisé par le fait que** le système de sonde comprend un appareil d'entrée de solution physiologique et une sonde à plasma, et un générateur de plasma basse température selon l'une des revendications précédentes qui est connecté à la sonde à plasma.

4. Système de sonde composé d'un générateur de plasma basse température selon la revendication 3, **caractérisé par le fait que** le module de sortie comprend :
un premier circuit d'amplification de puissance, dont l'extrémité d'entrée est connectée à la première unité de vibration ;
un second circuit d'amplification de puissance, dont l'extrémité d'entrée est connectée au circuit d'amplification de tension et qui entre des signaux de détection dans le circuit de détection de courant.

5. Système de sonde composé d'un générateur de plasma basse température selon la revendication 4, **caractérisé par le fait que** la sonde à plasma comprend un embout outil et une poignée qui sont fixés d'un seul tenant,
la poignée comprenant un corps de poignée (7), une interface d'entrée d'électrode qui est prévue sur le corps de poignée (7) et est connectée à l'extrémité de sortie du premier circuit d'amplification de puissance et du second circuit d'amplification de puissance, respectivement, et une interface d'entrée de solution physiologique qui est prévue sur une extrémité du corps de poignée (7) et un orifice d'évacuation de déchet liquide situé sur la même extrémité du corps de poignée (7) que l'interface d'entrée de solution physiologique ;
l'embout outil comprenant un corps d'embout outil (8) qui est fixé d'un seul tenant avec le corps de poignée (7), une électrode prévue sur le corps d'embout outil (8) et connectée à l'interface d'entrée d'électrode, l'électrode comprenant une première extrémité (9) de l'électrode prévue sur le corps d'embout outil (8), et une seconde extrémité (12) de l'électrode prévue à l'extrémité qui est à distance du corps de poignée (7) sur l'embout outil, et un trou de sortie de solution physiologique (10) prévu sur le corps d'embout outil (8) et en liaison mutuelle avec l'interface d'entrée de solution physiologique, et un trou d'absorption de déchet liquide (11) prévu à l'extrémité qui est à distance de la poignée sur le corps d'embout outil (8) et en liaison mutuelle avec l'orifice d'évacuation de déchet liquide.

6. Système de sonde composé d'un générateur de plasma basse température selon la revendication 5, **caractérisé par le fait que** l'appareil d'entrée de solution physiologique comprend une bouteille de stockage de solution physiologique (1) ; un tube de transfert (2), dont une extrémité est prévue sur la bouteille de stockage de solution physiologique (1) et en liaison mutuelle avec celle-ci ; et un dispositif de commande d'entrée de solution physiologique (3) prévu sur le tube de transfert (2), l'autre extrémité du tube de transfert (2) étant connectée à l'interface d'entrée de solution physiologique.
